# EUROPEAN PATENT APPLICATION

(11) **EP 3 318 187 A1**
(43) Date of publication of application: **09.05.2018**
(21) Application number: 16197453.0
(22) Date of filing: 07.11.2016
(51) Int. Cl.: A61B 5/0408, A61B 5/0478, A61N 1/04

(54) **ELECTRODE FOR PHYSIOLOGICAL MEASUREMENTS**

(71) Applicant: Nokia Technologies Oy, 02610 Espoo (FI)
(72) Inventor: KÄRKKÄINEN, Leo Mikko Johannes, 00100 Helsinki (FI); BLOMQVIST, Kim Henrik, 02650 Espoo (FI); HONKALA, Mikko, 02770 Espoo (FI)
(74) Representative: Nokia EPO representatives

(57) **Abstract**

An electrode for physiological measurements, the electrode comprising: a contact surface configured to provide contact to physiological substance, and at least two contact elements in the contact surface, wherein the contact elements are spatially separated from each other.

## Description

### TECHNICAL FIELD

The present application generally relates to physiological measurements, such as for example electrocardiogram (ECG) measurements and/or other biomedical/biosignal measurements, and to electrodes for such physiological measurements.

### BACKGROUND

This section illustrates useful background information without admission of any technique described herein representative of the state of the art.

Physiological measurements set certain requirements to measurement equipment. There are certain sources of errors in these measurements and the aim is to be able to take the error sources into account in the measurement or to avoid the errors.

### SUMMARY

Various aspects of examples of the invention are set out in the claims.

According to a first example aspect of the present invention, there is provided an electrode for physiological measurements, the electrode comprising:
a contact surface configured to provide contact to physiological substance, and
at least two contact elements in the contact surface, wherein the contact elements are spatially separated from each other.

In an embodiment the electrode comprises more than two contact elements and some of the contact elements are electrically coupled with each other. The coupling may be galvanic or through a multiplexer.

In an embodiment the electrode is configured to measure impedance between at least two contact elements. In an embodiment there is a current source connected between the at least two contact elements.

In an embodiment at least one of the contact elements is configured to provide a physiological measurement signal, such as for example measurement signals for electrocardiogram measurements.

In an embodiment layout of the contact elements on the contact surface fulfills one or more of the following criteria: the contact elements have a half circle like form, the contact elements have a pie like layout, the contact elements form a plurality of nested circles, and the contact elements are at least partially interlaced between each other or have a finger-like form.

In an embodiment the contact elements have equal size.

In an embodiment the electrode comprises liquid-resistant material between the contact elements.

According to a second example aspect of the present invention, there is provided a method comprising
sensing electrophysiological measurement signals from a physiological substance using an electrode that comprises
- a contact surface configured to provide contact to the physiological substance, and
- at least two contact elements in the contact surface, wherein the contact elements are spatially separated from each other.

In an embodiment, the method further comprises using electrophysiological measurement signal from at least one of the contact elements for electrophysiological measurement.

In an embodiment, the method further comprises measuring electrode impedance between at least two contact elements.

In an embodiment, the method further comprises using the impedance for processing electrophysiological measurement results obtained using the electrode. In an embodiment the processing comprises removing erroneous measurement results. In an embodiment the processing comprises de-noising the measurement results. In an embodiment the processing comprises stabilizing the measurement results with analog circuitry.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Figs. 1A-1B show certain measurement arrangements according to some embodiments of the invention;
Fig. 2 shows a circuit diagram according to an embodiment of the invention;
Figs. 3A-3B show certain measurement arrangements according to some embodiments of the invention;
Figs. 4-7 show some examples of electrode structures; and
Fig. 8 shows a flow diagram illustrating a method according to an embodiment of the invention.

### DETAILED DESCRIPTON OF THE DRAWINGS

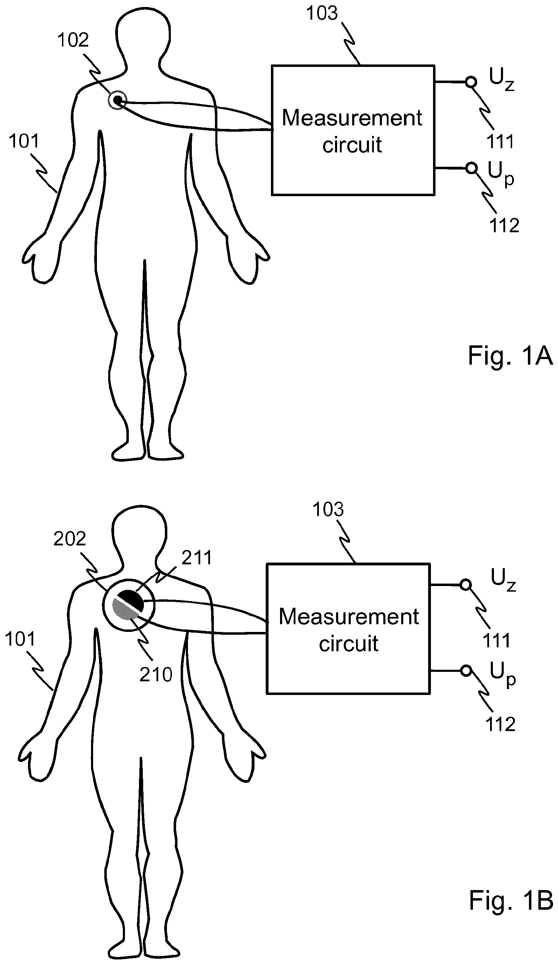

Example embodiments of the present invention and its potential advantages are understood by referring to Figs. 1 through 8 of the drawings. In this document, like reference signs denote like parts or steps.

In this document, the terms couple and connect may refer to direct contact between components or to coupling through some intervening component(s).

Various embodiment of the invention disclosed in the following relate to biomedical or physiological measurements. Herein, the term biomedical / physiological measurements is generally used to refer to electronic measurements of biomedical substance or organic material. The biomedical substance may be for example body or tissue of a living organism (e.g. human being) or a cell sample. Examples of biomedical measurements comprise for example electrocardiography (ECG) measurements, skin conductivity (aka electrodermal activity, EDA) measurements, body conductivity (aka electrical bioimpedance) measurements, and impedance plethysmography (IPG) such as impedance cardiography measurements (ICG).

In the following, various example embodiments are discussed in connection with ECG measurement topologies. Various embodiments are however not necessarily limited to ECG measurements only. Instead the example embodiments can be used other biomedical measurements, too.

Electrodes are used in physiological measurements for providing contact to the substance that is being measured (e.g. human body) to obtain the desired electrophysiological measurement signal. It is desirable to have consistent and accurate signal detection. E.g. motion artifacts cause challenges in the measurement (e.g. in ECG measurement). One source of motion artifacts is the partial detachment and subsequent reconnection of the electrode on the skin. Due to partial detachment the electrode-to-skin electrical impedance may vary considerably and cause errors in the measurement results.

Some implementations use wet or silicone electrode patches that are glued to the skin. It is believed that in some circumstances the wet electrodes may reduce detachment of the electrode, but the wet electrodes can be uncomfortable to the user especially with long measurement period. To improve user experience it is desirable to use a system that allows the skin to breath.

With non-glued electrodes e.g. dry electrodes (e.g. electrodes wound in a fabric or made of conductive plastic or foam, for example in a chest band) the skin is allowed to breath, but partial detachment of the electrode is more likely. In order to be able to obtain good measurement results despite of this, there is a desire to detect the electrode-to-skin resistance/impedance or changes in the electrode-to-skin resistance/impedance and to use the detected resistance/impedance changes to remove erroneous measurement data or to denoise noisy measurement data or to directly stabilize the measurement signal with a suitable analog circuitry.

In an embodiment of the invention there is provided a new intelligent electrode structure. The electrode comprises a split structure comprising at least two spatially insulated contact elements. The split electrode structure allows detection of electrode-to-skin resistance/impedance or changes in the electrode-to-skin resistance/impedance with only one electrode. That is, the structure allows measurement of the electrode contact resistance/impedance locally under single electrode without need to have a separate reference electrode. The split electrode structure allows detection of partial detachment of the electrode and thereby the detachment can be taken into account in final measurement results. With the split electrode structure one can provide an electrode that is able to report its own resistance/impedance (electrode-to-skin connectivity). This resistance/impedance can then be used to improve measurement results in a measurement circuit that processes signals sensed by the electrode. E.g. machine-learning techniques can be used for this purpose.

In an embodiment the contact elements of the electrode are spatially separated by an empty space between them. In another embodiment the electrode comprises hydrophobic liquid-resistant material between the contact elements to provide the insulation between the contact elements. The liquid-resistant material reduces exposure of the electrode for example to water and sweat. The liquid-resistant material may be liquid-impermeable material and may be referred to as water-resistant or water-repellent or waterproof material. There may be e.g. a liquid-resistant coating. Also some other material may be used for providing the insulation.

The split/distributed electrode structure with a plurality of (or at least two) contact elements is fundamentally different from electrodes where one electrode provides one measurement signal and if any kind of comparative measurements or reference signals are needed, an additional electrode is needed.

In case two separate electrodes were used for estimating electrode impedance one would not actually get results that would concern one single electrode. Instead one would only obtain an estimate illustrative of differences between the electrodes. Therefore structure that allows measurement based on single electrode provides benefits.

Fig. 1A shows a generalized measurement arrangement according to an embodiment of the invention. The shown arrangement comprises a human body 101 (i.e. the substance that is being measured), an electrode 102 attached to the human body 101, and a measurement circuit 103.

The electrode 102 is configured to provide contact to the human body 101 to obtain a measurement signal. In an embodiment the electrode 102 has a split electrode structure comprising at least two spatially insulated contact elements. Further, the electrode 102 is connected to the measurement circuit 103 to convey the measurement signal to the measurement circuit 103. Measurement signals from the contact elements may be connected to the measurement circuit 103 as separate signals or the electrode 102 may comprise circuitry (not shown) that combines the signals from the contact elements prior to connection to the measurement circuit 103.

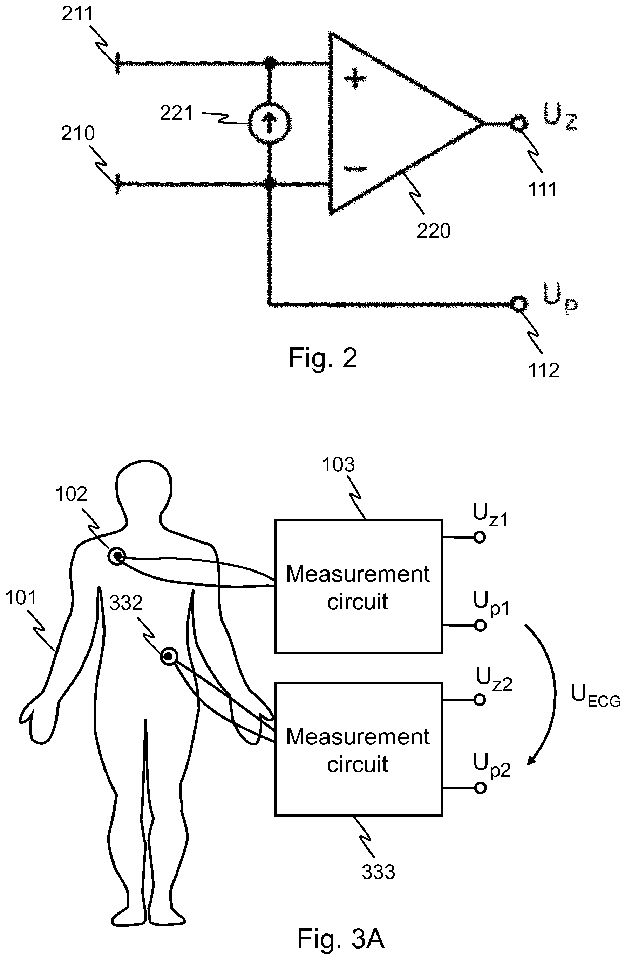

The measurement circuit 103 comprises suitable circuitry configured to process the measurement signal. The measurement circuit provides two outputs; an impedance output 111 and a physiological output 112. These outputs may be referred to as outputs of the electrode. The impedance output 111 of the measurement circuit 103 provides electrode impedance Uz and the physiological output 112 of the measurement circuit 103 provides a physiological measurement signal Up. That is, the actual measurement signal measured with the electrode is provided in physiological output 112 and the electrode output 111 reports impedance of the electrode 102. An example circuit 103 is shown in Fig. 2.

In an embodiment the electrode impedance Uz is used for de-noising the physiological measurement signal Up or the electrode impedance Uz may be otherwise used in processing of the physiological measurement signal Up. Furthermore, the outputs 111 and 112 of the measurement circuit may be combined with outputs of other electrodes to provide a desired result signal.

It is to be noted that the measurement circuit 103 is shown as an external separate component for visual purposes. In an actual physical implementation the measurement circuit 103 may be implemented in the electrode 102 or the measurement circuit may be part of a larger processing circuit.

Fig. 1B shows a generalized measurement arrangement according to an embodiment of the invention. Similarly to Fig. 1A the arrangement of Fig. 1 B comprises a human body 101 (i.e. the substance that is being measured), an electrode 202 attached to the human body 101, and a measurement circuit 103. The embodiment shown in Fig. 1B is similar to the arrangement of Fig. 1A except that Fig. 1B shows an expanded electrode 202 showing an example of a split electrode structure. The electrode 202 comprises two spatially insulated contact elements 210 and 211. Each of the contact elements 210 and 211 is separately connected to the measurement circuit 103 and the measurement circuit 103 is configured to perform suitable processing of the signals sensed by the contact elements 210 and 211.

One needs to note that the size of the electrode 202 in Fig. 1B is larger than the size of the electrode 102 in Fig. 1A purely for illustrative reasons. The shown size does not reflect the actual size of an electrode.

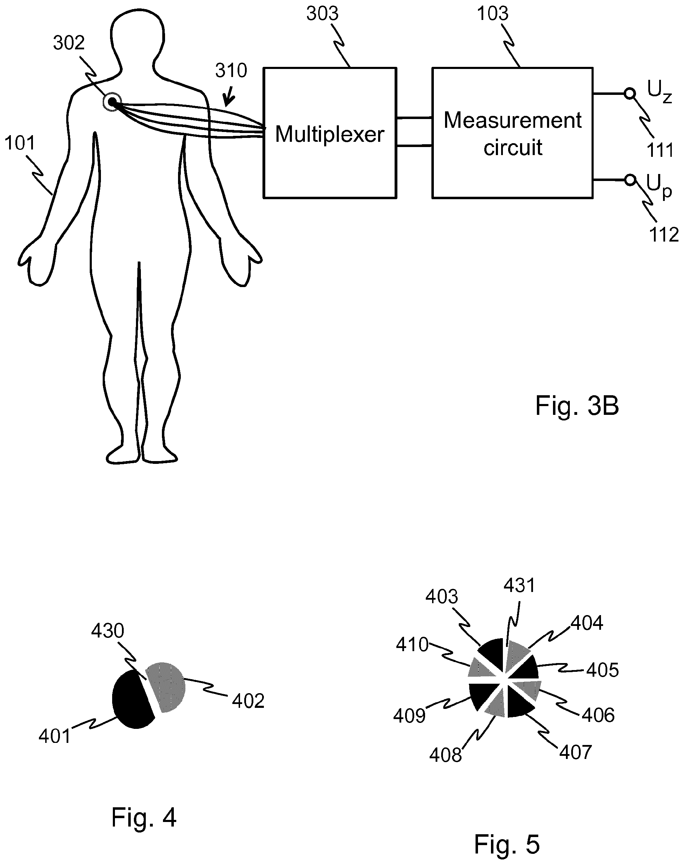

Fig. 2 shows a circuit diagram according to an embodiment of the invention. Contact elements (e.g. contact elements 210 and 211 of Fig. 2) are connected to input of the circuit and the circuit outputs the impedance output 111 and the physiological output 112. The signal sensed by the contact element 210 is connected to the physiological output 112 to provide the physiological measurement signal Up. Current source 221 is connected between the contact elements 210 and 211. The contact elements 210 and 211 are connected to an amplifier 220 and the amplifier 220 is configured to measure impedance between the contact elements 210 and 211. Output of the amplifier 220 is connected to the impedance output 111 to provide electrode impedance Uz. In this example embodiment Uz and Up are voltage outputs, while in Figs. 1A, 1B, 3A and 3B the Uz and Up output may alternatively be other signal types, e.g. current signals.

It is to be noted that in the example shown in Fig. 2, the measurement signal from only one contact element is used for the physiological measurement signal Up. However, it is possible that measurement signals from other contact elements are also used for this.

Fig. 3A shows a generalized measurement arrangement with two electrodes according to an embodiment of the invention. Similarly to Figs. 1A and 1B the arrangement of Fig. 3A comprises a human body 101 (i.e. the substance that is being measured), an electrode 102 attached to the human body 101, and a measurement circuit 103. Additionally, the arrangement of Fig. 3A comprises another electrode 332 and associated another measurement circuit 333. Internal structure of the electrodes 102 and 332 is not shown for the sake of clarity, but it is understood that the electrodes comprise a plurality of (at least two) contact elements. Both measurement circuits 103 and 333 comprise an output providing electrode impedance Uz1 and Uz2, respectively, and an output providing physiological measurement signal Up1 and Up2, respectively. Desired measurement result, such as for example ECG measurement signal, may be obtained over the physiological measurement signal Up1 and Up2. For the sake of clarity, a circuit arrangement used for obtaining the ECG signal from the physiological measurement signals Up1 and Up2 is not shown.

Fig. 3B shows a generalized measurement arrangement according to an embodiment of the invention. Similarly to Figs. 1A, 1B and 3A the arrangement of Fig. 3B comprises a human body 101 (i.e. the substance that is being measured), an electrode 302 attached to the human body 101, and a measurement circuit 103. Additionally, Fig. 3B shows a multiplexer circuit 303. Internal structure of the electrode 302 is not shown for the sake of clarity, but it is understood that the electrode 302 comprises a plurality of contact elements. There may be for example three or more contact elements in the electrode. Each of the contact elements is separately connected to the multiplexer circuit 303 through wiring 310. The multiplexer circuit 303 is configured to perform suitable combining / selection of the measurement signals sensed by the contact elements of the electrode 302 and to provide two outputs, one for the purpose of physiological measurement one for the purpose of electrode impedance measurement. The multiplexer circuit 303 may comprise for example two parallel N-to-2 multiplexer components that are configured to select desired measurement signals from the contact elements to the outputs of the multiplexer circuit 303. The outputs of the multiplexer circuit 303 are connected to inputs of the measurement circuit 103. The measurement circuit 103 is configured to perform suitable processing of the signals received from the multiplexer circuit 303.

It is to be noted that multiplexer circuit 303 is shown as an external separate component for visual purposes. In an actual physical implementation the multiplexer circuit 303 may be part of the measurement circuit 103 or implemented in the electrode 302. Or in some embodiments signals can be measured parallel without the multiplexer 103.

Additionally or alternatively, the arrangement may comprise circuitry (not shown) configured to amplify and buffer the measurement signals sensed by the contact elements, Uz and/or Up. The amplification and buffering may be implemented in in the electrode 302, in the multiplexer circuit 303 or in the measurement circuit 103. A benefit obtained by implementing the buffering in the electrode is that the buffer amplifier makes the wire low impedance, which may make the signals less prone to noise in the wires that connect the electrode 302 to the measurement circuit 103.

In an embodiment the electrode 102, 202, 302, 332 with a split contact structure is used also for ejecting current or voltage to the body 101. E.g. measurement current or voltage can be ejected in this way.

In an embodiment the split electrode structure refers to an electrode with distributed or interlaced contact element structure. There may be two or more contact elements.

In an embodiment there is even number of contact elements in the electrode. However, also odd number of contact elements can be used. In an embodiment, the size of the contact elements is equal whereby measurement signals sensed by the contact elements are directly comparable. However, also different sizes can be used. In that case for example suitable resistors can be used to allow sensible comparison of signals sensed by contact elements with different sizes.

Figs. 4-7 show some examples of electrode structures. One needs to note that also combinations of the shown example structures are possible. Figs. 4-7 show a contact surface of the example electrodes. The contact surface is configured to provide contact to physiological substance that is being measured.

Fig. 4 shows a two-fold patch with two contact elements 401, 402. The contact elements 401 and 402 are separated from each other by space 430. This structure allows detection of detachment of the patch in one direction.

Fig. 5 shows a pie like structure with eight contact element slices 403-410. The contact elements 403-410 are separated from each other by space 431. This structure allows detection of detachment of the patch from any direction. Also it is possible to wire each pair individually to have spatially better estimate. Here pair of contact elements refers to adjacent contact elements. In an embodiment, every other slice is used for the physiological signal measurement and every other slice is used for the impedance measurement. It is understood that also other number of pie slices may be used. There may be e.g. four slices.

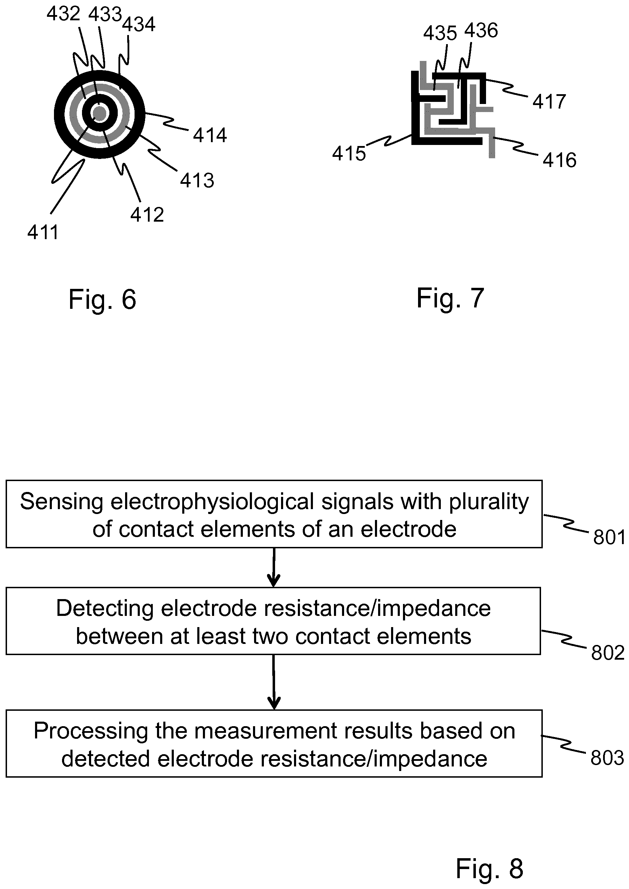

Fig. 6 shows a nested circle structure comprising contact elements 411-414. The circular contact elements 411-414 are separated from each other by circular spaces 432-434. This structure allows detection of detachment of the electrode patch in the middle of the electrode (a bubble-type detachment).

Fig. 7 shows an interlaced or fractal structure with contact elements 415 and 417 being interlaced with contact element 416. The contact elements 415-417 are separated from each other by spaces 435 and 436. The shown example comprises three separate contact elements, but in other examples there may be two interlaced contact elements or more than three interlaced contact elements.

The spaces 430-436 in Figs. 4-7 may be empty spaces or the spaces 430-436 may be filled with suitable material such as liquid-resistant material or other insulating material.

In an embodiment, some of the contact elements of an electrode are electrically coupled with each other. There may be e.g. galvanic coupling or the coupling may be through a multiplexer.

The electrode of various embodiments may be formed of layers of printed electronics components. In an example implementation there is a first (contact) layer comprising spatially separated contact elements. The first layer forms a contact surface configured to provide connection to the substance that is being measured. On top of the first layer there may be a second (connection) layer that comprises necessary components configured to enable measurement of mutual resistances/impedances between the contact elements. Further printed electronics may be used for implementing multiplexing and/or amplifying components.

Fig. 8 shows a flow diagram illustrating a method according to an embodiment of the invention.

Phase 801: Electrophysiological signals are sensed with a plurality of contact elements of one electrode.

Phase 802: Electrode resistances/impedances or variations thereof between at least two contact elements are detected.

Phase 803: The measurement results are processed based on the detected electrode resistances/impedances or variations thereof.

The processing in phase 803 may comprise one or more of the following: removing erroneous measurement results, de-noising the measurement results, stabilizing the measurement results with analog circuitry and otherwise cleaning the measurement results. Electrode impedance variations are causally connected to motion artifacts in ECG signals and therefore the electrode impedance variations are suited for cleaning the measurement results to provide desired clean result.

In the following, we discuss some examples of de-noising the measurement results (e.g. ECG signal) using the detected impedance variations:

### 1) De-noising using machine learning and a clean reference signal

A machine learning de-noising system is trained with ECG signal measured during physical activity of the person that is being measured. A first signal is recorded using glued electrodes, and this first signal is used as a clean reference signal. A second signal is recorded using a noisy dry electrode and also the electrode impedance (or electrode connectivity) is recorded. The machine learning system uses the first signal and the second signal as training signals to learn a regression model between the noisy dry electrode signal and the clean reference signal and by utilizing the electrode impedance information, too. After the model has been trained, the clean signal is no longer needed. The system can estimate the clean signal from the noisy dry electrode signal, if the electrode impedance or variations thereof are also known. Any machine learning regression model, such as neural networks, including Recurrent Neural Network and Convolutional Neural networks, may be used.

### 2) De-noising using machine learning and a noisy reference signal

This method is similar to option 1), except that the first signal is denoised before using it as the reference signal. The reason for this is that artifacts may exist even in the wet electrode signal. De-noising of the first signal can be done e.g., as a separate machine learning step that uses a de-noising autoencoder and a large number of existing clean samples (e.g., from hospital recordings, such as physionet MIMIC), which are noised, and a de-noising model is learned between the noised clean samples and the first signal.

### 3) De-noising using heuristic methods

In this method, covariance and correlation of the electrode connectivity signal and the noisy measurement signal are used as a noise signal that should be subtracted from the noisy measurement signal. Also the spectral components of the signals can be utilized here.

### 4) De-noising using analog circuit to match the electrode imbalance

In this method, the skin-electrode impedance imbalance is matched with a controlled resistance/reactance. Contact impedances of the contact elements are measured and the controlled resistance/reactance connecting the contact elements is tuned to match the imbalance. This will reject the differential noise voltage caused by motion (variation in the electrode contact impedances).

In an embodiment the detected electrode resistance/impedance is used for identifying problems with electrode attachment/electrode connection. The system may be configured to mark the measurement data, when the electrode is starting to detach. Then one does not have to analyze the corrupted data or save it or send it forward for further processing. Also the quality of the measurement results averages may be improved when bad/noisy data can be removed.

In another embodiment, the detected electrode resistance/impedance is used for tuning processing of the measurement results to improve the quality of signal. This may be done up to the point when the electrode fully detaches.

Without in any way limiting the scope, interpretation, or application of the claims appearing below, a technical effect of one or more of the example embodiments disclosed herein is that an improved measurement is provided. Measurement in less ideal circumstances may be enabled.

Another technical effect of one or more of the example embodiments disclosed herein is that one electrode patch suffices for the measurement arrangement. This results in improved user experience.

Yet another technical effect of one or more of the example embodiments disclosed herein is that the electrode structure of example embodiments may allow detection and alleviation of motion artifacts for better signal analysis.

The electrode of various embodiments may be well suited for example for ECG measurements and other physiological/biomedical measurements.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications, which may be made without departing from the scope of the present invention as defined in the appended claims.

## Claims

1. An electrode for physiological measurements, the electrode comprising:
a contact surface configured to provide contact to physiological substance, and
at least two contact elements in the contact surface, wherein the contact elements are spatially separated from each other.

2. The electrode of claim 1, wherein the electrode comprises more than two contact elements and some of the contact elements are electrically coupled with each other.

3. The electrode of claim 2, wherein the coupling is galvanic or through a multiplexer.

4. The electrode of any preceding claim, wherein the electrode is configured to measure impedance between at least two contact elements.

5. The electrode of any preceding claim, wherein at least one of the contact elements is configured to provide a physiological measurement signal.

6. The electrode of any preceding claim, wherein layout of the contact elements on the contact surface fulfills one or more of the following criteria: the contact elements have a half circle like form, the contact elements have a pie like layout, the contact elements form a plurality of nested circles, and the contact elements are at least partially interlaced between each other.

7. The electrode of any preceding claim, wherein the contact elements have equal size.

8. The electrode of any preceding claim, wherein the electrode comprises liquid-resistant material between the contact elements.

9. A method comprising
sensing electrophysiological measurement signals from a physiological substance using an electrode that comprises
- a contact surface configured to provide contact to the physiological substance, and
- at least two contact elements in the contact surface, wherein the contact elements are spatially separated from each other.

10. The method of claim 9, further comprising using electrophysiological measurement signal from at least one of the contact elements for electrophysiological measurement.

11. The method of claim 9 or 10, further comprising measuring electrode impedance between at least two contact elements.

12. The method of claim 11, further comprising using the impedance for processing electrophysiological measurement results obtained using the electrode.

13. The method of claim 12, wherein said processing comprises removing erroneous measurement results.

14. The method of claim 12 or 13, wherein said processing comprises de-noising the measurement results.

15. The method of any one of claims 12-14, wherein said processing comprises stabilizing the measurement results with analog circuitry.
